# EUROPÄISCHE PATENTSCHRIFT

(11) **EP 1 582 157 B1**
(45) Veröffentlichungstag und Bekanntmachung des Hinweises auf die Patenterteilung: **03.10.2007**
(21) Anmeldenummer: 05012958.4
(22) Anmeldetag: 14.04.2000
(51) Int. Cl.: A61B 17/32

(54) **Medizinisches Instrument zum Präparieren von Gewebe**
Medical instrument for preparing tissue
Instrument médical pour préparer des tissus

(30) Priorität: 29.04.1999 EP 99108380
(43) Veröffentlichungstag der Anmeldung: 05.10.2005
(62) Teilanmeldung aus: 00107946.6
(73) Patentinhaber: Karl Storz GmbH & Co. KG, 78532 Tuttlingen (DE)
(72) Erfinder: Lang, Dieter, 96342 Stockheim (DE); Anders, Fridolin, 78194 Immendingen-Zimmern (DE); Bacher, Uwe, 78532 Tuttlingen (DE)
(74) Vertreter: Heuckeroth, Volker

(56) Entgegenhaltungen:
- DE-A- 4 300 064
- DE-A- 19 520 717
- DE-U- 8 712 835
- US-A- 4 646 745
- US-A- 5 507 772

## Beschreibung

Die Erfindung betrifft ein medizinisches Instrument zum Präparieren von Gewebe im menschlichen oder tierischen Körper nach dem Oberbegriff des Anspruchs 1.

Ein derartiges Instrument ist aus der US 5,507,772 bekannt.

Bei einem Instrument zum Präparieren von Gewebe handelt es sich um ein Instrument zum Abtrennen und/oder Fassen von Gewebe, beispielsweise um eine Stanze, Schere oder Zange.

Derartige Instrumente werden im Rahmen der minimal-invasiven Chirurgie dazu verwendet, Gewebe im menschlichen oder tierischen Körper, üblicherweise unter endoskopischer Sichtkontrolle, abzutrennen. Dazu weisen derartige Instrumente einen lang erstreckten Schaft auf, an dessen distalem Ende zumindest ein bewegliches Werkzeug angeordnet ist, das üblicherweise mit einem weiteren beweglichen oder unbeweglichen Werkzeug am distalen Ende des Schafts zum Abtrennen des Gewebes zusammenwirkt. Zum Betätigen des zumindest einen beweglichen Werkzeuges weisen derartige Instrumente ferner am proximalen Ende des Schafts zumindest ein bewegliches Griffteil auf, das über ein auf Schub und/oder Zug arbeitendes axial bewegliches Kraftübertragungselement kraftschlüssig mit dem zumindest einen beweglichen Werkzeug verbunden ist, so dass eine Bewegung des beweglichen Griffteils vom Kraftübertragungselement in eine Bewegung des beweglichen Werkzeugs übertragen wird.

Insbesondere bei operativen Eingriffen im Hals-Nasen-Ohrenbereich werden Instrumente verwendet, deren Schaft zumindest eine Biegung aufweist, um das oder die Werkzeuge am distalen Ende an schwer zugängliche Stellen, beispielsweise in Nischen der Stirn- oder Kieferhöhle, bringen zu können.

Bei einem Instrument mit einem gebogenen Schaft, insbesondere, wenn der Biegeradius klein ist, stellt sich das Problem, die Kraft vom beweglichen Griffteil über das Kraftübertragungselement durch die Biegung hindurch auf das bewegliche Werkzeug zu übertragen. Dies ist durch den Wunsch nach schlanken, d.h. dünnschaftigen Instrumenten besonders erschwert.

Um sich an die Biegung anpassen zu können, muss das Kraftübertragungselement daher im Bereich der Biegung des Schafts eine Biegeelastizität aufweisen.

Aus der DE 44 44 025 Al ist beispielsweise ein Kraftübertragungselement für ein medizinisches Instrument in Form eines biegeelastischen Drahtelements offenbart, das sich an eine derartige Biegung anpassen kann. Ein solcher flexibler Draht kann jedoch nur Zugkräfte übertragen, ist jedoch nicht geeignet, Schubkräfte, insbesondere hohe Schubkräfte, zu übertragen. Wird das Kraftübertragungselement nämlich auf Schub belastet, knickt der Draht aus, so daß nur eine geringe oder keine Schubkraft auf das zumindest eine bewegliche Werkzeug übertragen werden kann, um dieses zum Abtrennen von Gewebe zu betätigen.

Da mit derartigen Instrumenten unter anderem auch hartes Gewebe, beispielsweise Knorpel- oder Knochengewebe, abgetrennt werden soll, muss das Kraftübertragungselement daher so beschaffen sein, dass auch sehr hohe Kräfte, insbesondere hohe Schubkräfte, auf das bewegliche Werkzeug übertragen werden können, ohne dass das Kraftübertragungselement dabei ausknickt, wobei die Kraftübertragung andererseits wohlgemerkt über die Biegung des Schafts erfolgen muss, das Kraftübertragungselement sich bei seiner axialen Bewegung also dem Biegungsverlauf anpassen können muss.

Die DE 195 20 717 C2 offenbart zur Lösung dieses Problems ein Rohrschaftinstrument mit einem rohrförmigen Schaft, der eine Biegung aufweist, wobei das Kraftübertragungselement im Innern des Rohrschafts angeordnet ist. Das Kraftübertragungselement ist im gebogenen Bereich des Rohrschafts als Stab aus einem biegeelastischen Material ausgebildet, der an der Innenwand einer ihn umgebenden starren, konzentrisch zum Schaft verlaufenden Hülse anliegt, und dessen Querschnitt im Bereich der Biegung durch eine Anzahl von axial nebeneinander angeordneten Umfangsnuten bereichsweise reduziert ist. Die zuvor genannte Hülse kann dabei auch durch den Rohrschaft selbst gebildet sein. Die Umfangsnuten sind an dem Stab voll umfänglich ausgebildet. Im Bereich der Umfangsnuten ist der Stab somit drahtförmig verdünnt mit rundem Querschnitt ausgebildet. In den Zwischenbereichen zwischen den Nuten ist der Durchmesser des Stabs nicht verringert, so dass die Außenflächen der Zwischenbereiche als Führungsflächen dienen, um das Kraftübertragungselement im Bereich der Biegung an der Innenwand der Hülse bzw. des Rohrschaftes zu führen.

Mit einem solchen Kraftübertragungselement lassen sich zwar hohe Schubkräfte durch die Biegung hindurch auf das bewegliche Werkzeug übertragen, dennoch kann dieses bekannte Instrument mit dieser Ausgestaltung des Kraftübertragungselements als nachteilig angesehen werden.

Da derartige Instrumente im Rahmen der minimal-invasiven Chirurgie verwendet werden, besteht nämlich eine weitere Anforderung an derartige Instrumente darin, dass ihr Durchmesser im Bereich des Schaftes möglichst klein ist, um das Instrument mit dem Schaft durch eine möglichst kleine Inzision oder natürliche Körperöffnung in den Körper einführen zu können. Bei derartigen miniaturisierten Instrumenten mit einem Schaftdurchmesser von wenigen Millimetern bedeutet dies für das bekannte Instrument, dass das Kraftübertragungselement in den verdünnten Bereichen der Umfangsnuten einen sehr kleinen Durchmesser aufweist, wodurch die Stabilität des Kraftübertragungselements verringert ist, da sich die Materialstärke des Kraftübertragungselements im Bereich der Umfangsnuten auf sehr dünne Drahtabschnitte reduziert. Im Bereich der Umfangsnuten kann es daher bei der Übertragung hoher Schubkräfte oder bei stoßartigen Schubkräften zu einem Knicken, Brechen oder bei Übertragung hoher Zugkräfte zu einem Reißen des Kraftübertragungselements im Bereich der vollumfänglich ausgebildeten Umfangsnuten kommen, wodurch die Betriebssicherheit des bekannten Instruments verringert ist.

Die DE 43 00 064 Al offenbart eine Gewebestanze mit einem Außenschaft und einem Innenschaft, an dessen distalem Ende eine Öffnung mit einer Schneide vorgesehen ist, die mit einer Gegenschneide am distalen Ende des Außenschaftes so zusammenarbeitet, daß bei Betätigung der Gewebestanze Gewebe, das durch die erwähnte Öffnung in den Innenschaft ragt, durch die gegeneinander bewegten Schneiden abgetrennt wird. Der Innenschaft ist starr ausgebildet, verläuft im proximalen Bereich gerade und geht distalwärts in einen gekrümmten Verlauf über, und der Außenschaft, der das Kraftübertragungselement dieses Instruments bildet, ist zumindest im Bereich dieser Krümmung verformbar. Die Verformbarkeit des Außenschaftes wird dadurch ermöglicht, dass dieser mit Ausnehmungen versehen ist, die sich auf den Radien der Krümmung des Innenschaftes gegenüberliegen. Die Ausnehmungen in Form von radialen Einschnitten belassen lediglich eine schmale Materialbrücke zwischen sich.

Die bereits genannte US 5,507,772 offenbart ebenfalls ein medizinisches Instrument, dessen Schaft eine Biegung aufweist. Das Kraftübertragungselement weist eine Mehrzahl von eingekerbten oder ausgesparten Abschnitten im Bereich der Biegung des Schafts auf. Zwischen den ausgesparten Abschnitten des Kraftübertragungselements sind eine Mehrzahl von Rippen ausgebildet, deren Dicke im Wesentlichen der Dicke des übrigen Körpers des Kraftübertragungselements entspricht. Die Rippen sind nur zu einer Seite des Kraftübertragungselements hin ausgebildet, während das Kraftübertragungselement auf der den Rippen gegenüberliegenden Seite im Bereich der Biegung durchgehend flach ausgebildet ist. Das Kraftübertragungselement und dessen Rippen sind in einem länglichen Schlitz innerhalb des Schafts geführt, der in der konkaven Innenseite der Biegung des Schafts vorhanden ist. In einem anderen Ausführungsbeispiel sind die Rippen weggelassen, so dass das Kraftübertragungselement in diesem Fall im Bereich der Biegung als flaches Band ausgebildet ist, das dann aber nicht geführt ist.

Aus der US 4,646,745 ist weiterhin ein medizinisches Klammerinstrument bekannt, das ein in einem Schaft angeordnetes Kraftübertragungselement aufweist, wobei das Kraftübertragungselement in Form eines Flachbandes ausgebildet ist, so dass das Kraftübertragungselement flexibel ist. Das Flachband selbst besteht aus drei Lagen von dünnen Bändern. Im Bereich einer Biegung des Schafts ist das Flachband entlang eines Abstandselements geführt, um zu erreichen, dass das Flachband im Bereich der Biegung seine zentrische Lage im Schaft beibehält, wenn es unter Zugspannung steht, d.h. sich nicht in der Biegung des Schafts gerade zieht. Das Abstandselement ist mit Kugellagern zur Verminderung der Reibung am Kraftübertragungselement versehen.

Der Erfindung liegt die Aufgabe zugrunde, ein medizinisches Instrument der eingangs genannten Arten dahingehend weiterzubilden, dass mittels des Kraftübertragungselements über die Biegung des Schaftes hinweg hohe Zug- und/oder Schubkräfte auf das zumindest eine bewegliche Werkzeug übertragen werden können, ohne dass das Kraftübertragungselement dabei ausknickt, und ohne dass im Bereich der Biegung eine Verringerung der Stabilität des Kraftübertragungselements auftritt.

Erfindungsgemäß wird die der Erfindung zugrunde liegende Aufgabe hinsichtlich des eingangs genannten medizinischen Instruments dadurch gelöst, dass das Kraftübertragungselement im Bereich der Biegung axial durchgehend als flaches Band ausgebildet ist, das mit seinen Schmalseiten in seitlichen Führungen des Schafts geführt ist.

Diese Ausgestaltung hat den Vorteil, dass die flexible Ausgestaltung des Kraftübertragungselements im Bereich der Biegung des Schafts konstruktiv und fertigungstechnisch besonders einfach ist. Durch die Führung der Schmalseiten in seitliche Führungen des Schafts wird auch hier ein Ausknicken des Kraftübertragungselements im Bereich der Biegung vermieden.

Dabei ist es bevorzugt, wenn die Führungen als Nuten in einer Innenwand des Schafts ausgebildet sind.

Hierbei ist von Vorteil, dass der Schaft im Bereich der Biegung geschlossen ausgebildet werden kann, wodurch das Eindringen von Verunreinigungen in den Schaft vermieden wird.

Alternativ dazu ist es jedoch auch bevorzugt, wenn die Führungen als offene Schlitze in dem Schaft ausgebildet sind.

Diese Maßnahme hat nun den Vorteil, dass die Schlitze fertigungstechnisch besonders einfach in den Schaft eingebracht werden können, wodurch der Herstellungsaufwand des Instruments bei dieser Ausgestaltung verringert ist.

In einer weiteren bevorzugten Ausgestaltung dieses Instruments ist das Kraftübertragungselement in Bereichen außerhalb der Biegung als massiver Stab aus einem Vollmaterial ausgebildet.

Hierbei ist von Vorteil, dass das Kraftübertragungselement in Bereichen außerhalb der zumindest einen Biegung, insbesondere über sich lang erstreckende gerade Bereiche des Schafts besonders stabil und starr ausgebildet werden kann, wodurch sich das Kraftübertragungselement besonders für die Übertragung hoher Schubkräfte eignet.

In einer weiteren bevorzugten Ausgestaltung ist das flache Band in einem materialabtragenden Verfahren aus dem Stab aus Vollmaterial gebildet.

Hierbei ist von Vorteil, dass das flache Band und der übrige Körper des Kraftübertragungselements in einstückiger Weise miteinander verbunden sind, wobei Sollbruchstellen, wie sie beispielsweise Löt-, Schweiß- oder Klebstellen darstellen können, vermieden werden.

Alternativ dazu ist es jedoch auch bevorzugt, wenn das Band stoffschlüssig mit dem Stab verbunden ist.

Dies ist von Vorteil, wenn es aus fertigungstechnischen Gründen sinnvoll ist, Stab und Band in getrennten Arbeitsverfahren herzustellen.

In einer weiteren bevorzugten Ausgestaltung ist das zumindest eine bewegliche Werkzeug relativ zum Schaft axial beweglich, wobei ein zweites Werkzeug am distalen Ende des Schafts angeordnet ist, wobei das bewegliche Werkzeug und das zweite Werkzeug durch axiale Relativbewegung zueinander in Form einer Stanze zusammenwirken.

Eine solche Ausgestaltung hat insbesondere in sehr beengten Operationsgebieten, beispielsweise in der Stirn- oder Kieferhöhle, den Vorteil, dass die beiden Werkzeuge zum Abtrennen von Gewebe keinen über den Umfang des Schafts hinausgehenden freien Arbeitsradius benötigen, weil beide Werkzeuge lediglich axial in Längsrichtung des Schafts, genauer gesagt in Längsrichtung des distalen Endes des Schafts, relativ zueinander bewegt werden.

Dabei ist es weiterhin bevorzugt, wenn das zweite Werkzeug unbeweglich mit dem Schaft verbunden ist und distal vor dem beweglichen Werkzeug angeordnet ist.

Hierbei ist von Vorteil, dass das zweite unbewegliche Werkzeug die distale Spitze des Schafts bildet und beim Ansetzen dieses unbeweglichen Werkzeugs an dem abzutrennenden Gewebe einen ortsfesten Arbeitspunkt definiert. In diesem Fall arbeitet das Kraftübertragungselement auf Schub, um das bewegliche Werkzeug nach distal gegen das unbewegliche Werkzeug zu schieben, um zwischen dem unbeweglichen Werkzeug und dem beweglichen Werkzeug befindliches Gewebe abzutrennen, und auf Zug, um beide Werkzeuge wieder voneinander zu trennen.

In einer weiteren bevorzugten Ausgestaltung ist das zumindest eine bewegliche Werkzeug gegenüber dem zweiten Werkzeug verdrehgesichert.

Diese Maßnahme ist insbesondere im Falle des medizinischen Instruments von Vorteil, bei dem das Kraftübertragungselement als Rohr und im Bereich der Biegung als Schraubenfeder ausgebildet ist. Die Verdrehsicherung hält das bewegliche Werkzeug und das zweite Werkzeug in Drehrichtung um die Längsachse des Schafts lagefest zueinander, auch wenn auf die Schraubenfeder Torsionskräfte wirken sollten.

Alternativ zu der Ausgestaltung des zumindest einen beweglichen Werkzeuges als Stanzwerkzeug lässt sich die Erfindung jedoch vorteilhaft einsetzen, wenn das zumindest eine bewegliche Werkzeug als um ein Drehgelenk verschwenkbares Maulteil ausgebildet ist, das mit einem zweiten Werkzeug in der Art einer Schere oder Zange zusammenwirkt.

Weitere Vorteile ergeben sich aus der nachfolgenden Beschreibung und der beigefügten Zeichnung.

Es versteht sich, dass die vorstehend genannten und nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf diese näher beschrieben. Es zeigen:
- Fig. 1: ein Ausführungsbeispiel eines erfindungsgemäßen medizinischen Instruments in einer Gesamtdarstellung in Seitenansicht;
- Fig. 2: das distale Ende des Instruments in Fig. 1 in Seitenansicht und vergrößertem Maßstab;
- Fig. 3: eine der Fig. 2 entsprechende Darstellung im Längsschnitt; und
- Fig. 4: einen Schnitt entlang der Linie IV-IV in Fig. 3.

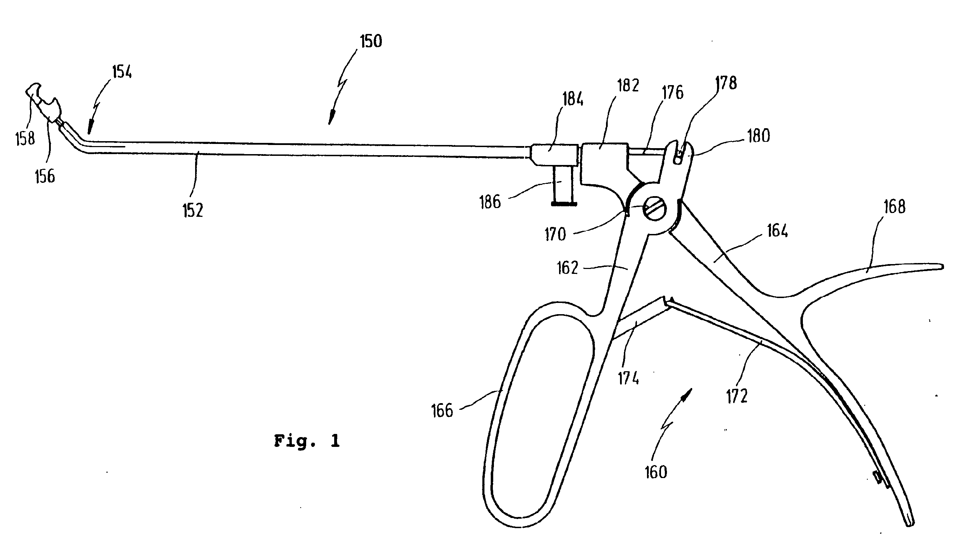

In Fig. 1 bis 4 ist ein Ausführungsbeispiel eines mit dem allgemeinen Bezugszeichen 150 versehenen medizinischen Instruments zum Abtrennen von Gewebe im menschlichen oder tierischen Körper dargestellt.

Das Instrument 150 weist einen lang erstreckten Schaft 152 auf, der eine Biegung 154 aufweist.

Am distalen Ende des Schafts 152 ist ein bewegliches Werkzeug 156 angeordnet, das axial beweglich ist und mit einem unbeweglichen Werkzeug 158, das ebenfalls am distalen Ende des Schafts 152 angeordnet ist, in der Art einer Stanze zusammenwirkt.

Am proximalen Ende des Schafts 152 ist eine Handhabe 160 mit einem beweglichen Griffteil 162 und einem unbeweglichen Griffteil 164 angeordnet.

Das bewegliche Griffteil 162 weist einen Ring 166 auf, durch das ein, zwei oder drei Finger einer Hand zur Betätigung des beweglichen Griffteils 162 durchgesteckt werden.

Das unbewegliche Griffteil 164 weist einen nach proximal abstehenden Fortsatz 168 auf, an dem sich beim Greifen der Handhabe 160 die Mulde zwischen Daumen und Zeigefinger abstützt.

Das bewegliche Griffteil 162 und das unbewegliche Griffteil 164 sind über ein Drehgelenk 170 gelenkig miteinander verbunden.

Eine Blattfeder 172, die an dem unbeweglichen Griffteil 164 befestigt ist, ist mit einem Hebel 174 verbunden, der an dem beweglichen Griffteil 162 angelenkt ist. Die Blattfeder 172 spannt im Zusammenwirken mit dem Hebel 174 das bewegliche Griffteil 164 in seine in Fig. 1 dargestellte Offenstellung vor, in der das bewegliche Griffteil 162 von dem unbeweglichen Griffteil 164 maximal beabstandet ist.

Das bewegliche Werkzeug 156 ist mit dem beweglichen Griffteil 162 durch ein Kraftübertragungselement 176 kraftschlüssig verbunden, das sich durch den Schaft 152 hindurch erstreckt.

Am äußersten proximalen Ende weist das Kraftübertragungselement 176 einen sich quer zur Längsrichtung des Kraftübertragungselements 176 erstreckenden Zapfen 178 auf, der in einen Gabelabschnitt 180 des beweglichen Griffteils 162 eingreift, wodurch das Kraftübertragungselement 176 kraftschlüssig mit dem beweglichen Griffteil 162 verbunden ist.

Das Kraftübertragungselement 176 ist dabei durch ein mit dem unbeweglichen Griffteil 164 fest verbundenes Rohrstück 182 durchgeführt, an dem der Schaft 152 über ein weiteres Rohrstück 184 unbeweglich befestigt ist.

An dem Rohrstück 184 ist ein Anschluss 186 zum Anschließen einer nicht dargestellten Spülleitung angeordnet, so dass durch den Anschluss 186 eine Spülflüssigkeit in den Schaft 152 eingeleitet werden kann, die durch den Schaft 152 zu dessen distalem Ende hin geleitet wird, wo sie dann in das Operationsgebiet austritt.

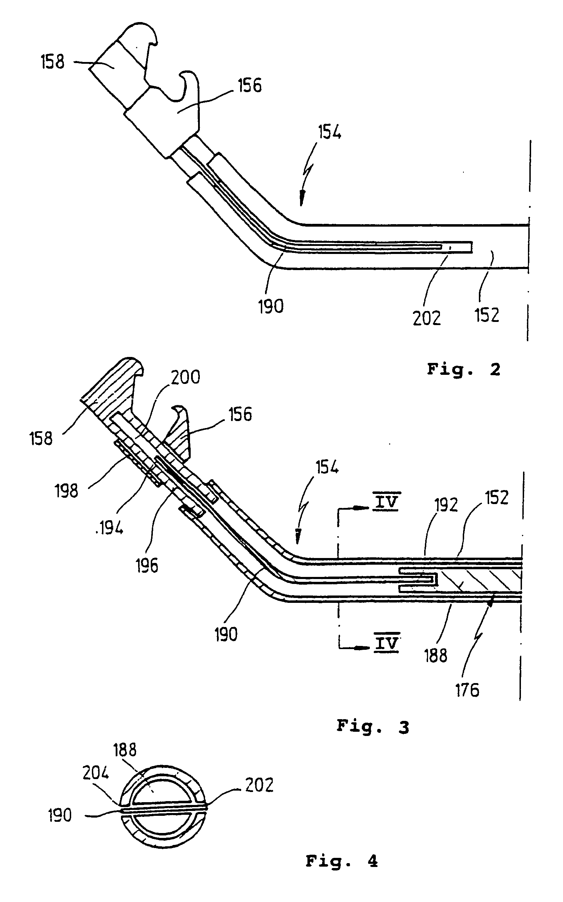

Das Kraftübertragungselement ist gemäß Fig. 3 im Bereich zwischen dem beweglichen Griffteil 162 und der Biegung 154 als Stab 188 ausgebildet. Der Stab 188 ist im Querschnitt rund und liegt am Schaft 152 an und ist in diesem bei seiner axialen Hin- und Herbewegung geführt.

Im Bereich der Biegung 154 ist das Kraftübertragungselement 176 durchgehend als flaches Band 190 ausgebildet, das biegeelastisch ist. Das flache Band 190 kann beispielsweise in Form einer Blattfeder aus einem Federstahl ausgebildet sein.

Das flache Band ist dabei so orientiert, daß die Flächennormale ihrer breiten Flachseite in der Krümmungsebene der Biegung 154 liegt, sich die flachen Breitseiten des Bandes 190 demnach in Richtung quer zur Krümmungsebene erstrecken. Durch diese Anordnung des flachen Bandes 190 ist das Kraftübertragungselement 176 im Bereich der Biegung in der Krümmungsebene der Biegung 154 flexibel und kann sich somit an den Verlauf der Biegung 154 stets anpassen.

Ein proximales Ende 192 des Bandes 190 ist dabei stoffschlüssig mit dem Stab 188 des Kraftübertragungselements 176 verbunden, beispielsweise durch Laserschweißen. Es kann jedoch auch vorgesehen sein, das flache Band 190 aus dem Stab 188 aus Vollmaterial durch ein materialabtragendes Verfahren zu bilden, wodurch eine einstückige Verbindung des flachen Bandes 190 mit dem Stab 188 erreicht wird.

Ein distales Ende 194 des flachen Bandes 190 ist stoffschlüssig mit dem beweglichen Werkzeug 156 verbunden, wodurch die kraftschlüssige Verbindung des Kraftübertragungselements 176 mit dem beweglichen Werkzeug 156 hergestellt wird.

Das bewegliche Werkzeug 156 sitzt axial verschiebbar auf dem unbeweglichen Werkzeug 158, das dazu einen rohrförmigen Fortsatz 196 aufweist, auf dem ein Hülsenabschnitt 198 des beweglichen Werkzeugs 156 im Gleitsitz angeordnet ist.

Der rohrförmige Fortsatz 196 des unbeweglichen Werkzeugs 158 ist seinerseits fest mit dem distalen Ende des Schafts 152, beispielsweise durch Schweißen, verbunden. In dem rohrförmigen Fortsatz 196 ist ferner ein seitlich durchgehender Schlitz 200 vorhanden, in dem das flache Band 190 axial verschiebbar aufgenommen ist.

Im Bereich der Biegung 154 weist der Schaft 152 zwei in der Schaftwand ausgebildete Führungen 202 und 204 in Form von Schlitzen auf, die durch die Schaftwand 152 durchgehen, so dass der Schaft 152 im Bereich der Führungen 202 und 204 offen ist.

Das flache Band 190 erstreckt sich quer zur Längsrichtung des Schafts 152 nicht nur über den Innendurchmesser, sondern auch bis über den gesamten Außendurchmesser des Schafts 152, so dass seine Schmalseiten in den Führungen 202 und 204 eingreifen, wodurch das flache Band 190 in den Führungen 202 und 204 entlang der Biegung 154 des Schafts 152 geführt ist.

## Patentansprüche

1. Medizinisches Instrument zum Präparieren von Gewebe im menschlichen oder tierischen Körper, mit einem Schaft (152), mit zumindest einem beweglichen Werkzeug (156) an einem distalen Ende des Schafts (152), mit zumindest einem beweglichen Griffteil (162) an einem proximalen Ende des Schafts (152), und mit einem sich längs des Schafts (152) erstreckenden, relativ zu diesem beweglichen auf Schub und/oder Zug arbeitenden Kraftübertragungselement (176), dessen proximales Ende mit dem zumindest einen beweglichen Griffteil (162) und dessen distales Ende mit dem zumindest einen beweglichen Werkzeug (156) kraftschlüssig verbunden ist, wobei der Schaft (152) an zumindest einer Stelle zwischen dem distalen Ende und dem proximalen Ende eine Biegung (154) aufweist, wobei das Kraftübertragungselement (176) zumindest im Bereich der Biegung (154) quer zu einer Krümmungsebene der Biegung (154) axial durchgehend als flaches biegeelastisches Band ausgebildet ist, das sich im Bereich der Biegung (154) in Richtung quer zur Krümmungsebene axial durchgehend zumindest über den Innendurchmesser des Schafts (152) erstreckt, **dadurch gekennzeichnet, dass** das flache Band (190) mit seinen Schmalseiten in seitlichen Führungen (202) des Schafts (152) geführt ist.

2. Instrument nach Anspruch 1, **dadurch gekennzeichnet, dass** die Führungen (202) als Nuten in einer Innenwand des Schafts (152) ausgebildet sind.

3. Instrument nach Anspruch 1, **dadurch gekennzeichnet, dass** die Führungen (202) als offene Schlitze (200) in dem Schaft (152) ausgebildet sind.

4. Instrument nach einem der Ansprüche 1 bis 3, **dadurch gekennzeichnet, dass** das Kraftübertragungselement (176) in Bereichen außerhalb der Biegung (154) als massiver Stab (188) aus einem Vollmaterial ausgebildet ist.

5. Instrument nach Anspruch 4, **dadurch gekennzeichnet, dass** das flache Band (190) in einem materialabtragenden Verfahren aus dem Stab (188) aus Vollmaterial gebildet ist.

6. Instrument nach Anspruch 4, **dadurch gekennzeichnet, dass** das Band (190) stoffschlüssig mit dem Stab (188) verbunden ist.

7. Instrument nach einem der Ansprüche 1 bis 6, **dadurch gekennzeichnet, dass** das zumindest eine bewegliche Werkzeug (156) relativ zum Schaft (152) axial beweglich ist, wobei ein zweites Werkzeug (158) am distalen Ende des Schafts (152) angeordnet ist, wobei das bewegliche Werkzeug (156) und das zweite Werkzeug (158) durch axiale Relativbewegung zueinander in Form einer Stanze zusammenwirken.

8. Instrument nach Anspruch 7, **dadurch gekennzeichnet, dass** das zweite Werkzeug (158) unbeweglich mit dem Schaft (152) verbunden ist und distal vor dem beweglichen Werkzeug (156) angeordnet ist.

9. Instrument nach Anspruch 7 oder 8, **dadurch gekennzeichnet, dass** das bewegliche Werkzeug (156) relativ zum zweiten Werkzeug (158) verdrehgesichert ist.

10. Instrument nach einem der Ansprüche 1 bis 9, **dadurch gekennzeichnet, dass** das zumindest eine bewegliche Werkzeug als um ein Drehgelenk verschwenkbares Maulteil ausgebildet ist, das mit einem zweiten Werkzeug in der Art einer Schere oder Zange zusammenwirkt.

## Claims

1. A medical instrument for dissecting tissue in the human or animal body, comprising a shaft (152), at least one movable tool (156) at a distal end of the shaft (152), at least one movable grip element (162) at a proximal end of the shaft (152), and a force transmission element (176) that extends along the shaft (152) and is movable relative to the latter and is operable for transmitting push and/or pull forces, and whose proximal end is connected to the at least one movable grip element (162) in force-locking fashion and whose distal end is connected to the at least one movable tool (156) in force-locking fashion, the shaft (152) having a bend (154) at at least one location between the distal end and the proximal end, wherein the force transmission element is configured, at least in the area of the bend (154) and transversely to a plane of curvature of the bend and continuously in axial direction, as flattened flexible band, which extends, in the region of the bend (154) in direction transversely to the plane of curvature and axially continuously at least over the inner diameter of the shaft (152), **characterized in that** the flattened band (190) is guided in lateral guides (202) of the shaft (152) via its narrow sides.

2. The instrument of claim 1, **characterized in that** the guides (202) are configured as grooves in an inner wall of the shaft (152).

3. The instrument of claim 1, **characterized in that** the guides (202) are configured as open slots (200) in the shaft (152).

4. The instrument of anyone of claims 1 through 3, **characterized in that** the force transmission element (176) is configured as a solid bar (188) from a solid material in regions outside the bend (154).

5. The instrument of claim 4, **characterized in that** the flattened band (190) is formed from the bar (188) of solid material in a machining method.

6. The instrument of claim 4, **characterized in that** the band (190) is connected with the bar (188) by a substance bond.

7. The instrument of anyone of claims 1 through 6, **characterized in that** the at least one movable tool (156) is axially movable relative to the shaft (152), wherein a second tool (158) is arranged at the distal end of the shaft (152), wherein the movable tool (156) and the second tool (158) co-act in the way of a punch by an axial relative movement with respect to one another.

8. The instrument of claim 7, **characterized in that** the second tool (158) is connected with the shaft (152) in immovable fashion and is arranged distally of the movable tool (156).

9. The instrument of claim 7 or 8, **characterized in that** the movable tool (156) is protected from torsion relative to the second tool (158).

10. The instrument of anyone of claims 1 through 9, **characterized in that** the at least one movable tool is configured as a jaw part which can be pivoted about a hinge joint and which co-acts with the second tool in the manner of a pair of scissors or a forceps.

## Revendications

1. Instrument médical pour la préparation de tissu dans l'organisme humain ou animal, avec une tige (152), avec au moins un outil mobile (156) à une extrémité distale de la tige (152), avec au moins une partie de poignée mobile (162) à une extrémité proximale de la tige (152), et avec un élément de transmission de force (176) s'étendant le long de la tige (152), mobile par rapport à celle-ci et travaillant en poussée et/ou en traction, dont l'extrémité proximale est reliée selon une liaison positive à ladite au moins une partie de poignée mobile (162) et l'extrémité distale audit au moins un outil mobile (156), la tige (152) présentant à un endroit au moins entre l'extrémité distale et l'extrémité proximale une courbure (154), l'élément de transmission de force (176) étant réalisé, au moins dans la zone de la courbure (154) qui est perpendiculaire à un plan de courbure de la courbure (154), de façon axialement continue, sous la forme d'une bande plate flexible élastiquement s'étendant, dans la zone de la courbure (154) dans la direction perpendiculaire au plan de courbure, de façon axialement continue, au moins sur le diamètre intérieur de la tige (152), **caractérisé en ce que** la bande plate (190) est guidée, par ses chants, dans des éléments de guidage (202) latéraux de la tige (152).

2. Instrument selon la revendication 1, **caractérisé en ce que** les éléments de guidage (202) sont conformés en rainures ménagées dans une paroi intérieure de la tige (152).

3. Instrument selon la revendication 1, **caractérisé en ce que** les éléments de guidage (202) sont conformés en fentes ouvertes (200) ménagées dans la tige (152).

4. Instrument selon l'une des revendications 1 à 3, **caractérisé en ce que** l'élément de transmission de force (176), dans des zones situées en-dehors de la courbure (154), est conformé en baguette massive (188) en matériau plein.

5. Instrument selon la revendication 4, **caractérisé en ce que** la bande plate (190) est formée, suivant un procédé d'enlèvement de matière, à partir de la baguette (188) en matériau plein.

6. Instrument selon la revendication 4, **caractérisé en ce que** la bande (190) est reliée avec une continuité de la matière, à la baguette (188).

7. Instrument selon l'une des revendications 1 à 6, **caractérisé en ce que** ledit au moins un outil mobile (156) est mobile axialement par rapport à la tige (152), un deuxième outil (158) étant disposé à l'extrémité distale de la tige (152), l'outil mobile (156) et le deuxième outil (158) coopérant par un mouvement axial relatif l'un par rapport à l'autre à la manière d'une poinçonneuse.

8. Instrument selon la revendication 7, **caractérisé en ce que** le deuxième outil (158) est relié de manière immobile à la tige (152) et disposé distalement avant l'outil mobile (156).

9. Instrument selon la revendication 7 ou 8, **caractérisé en ce que** l'outil mobile (156) est verrouillé en torsion par rapport au deuxième outil (158).

10. Instrument selon l'une des revendications 1 à 9, **caractérisé en ce que** ledit au moins un outil mobile est réalisé sous la forme d'une partie de mâchoire, pouvant pivoter autour d'une articulation tournante, qui coopère avec un deuxième outil à la manière d'une cisaille ou d'une pince.
